# EUROPEAN PATENT SPECIFICATION

(11) **EP 4 217 061 B1**
(45) Date of publication and mention of the grant of the patent: **11.09.2024**
(21) Application number: 21773639.6
(22) Date of filing: 20.09.2021
(51) Int. Cl.: A61P 17/00, A61K 36/38, A61K 36/58

(54) **HYPERICUM EXTRACT IN NEEM OIL FOR THE TREATMENT OF EPIDERMOLYSIS BULLOSA**
HYPERICUM-EXTRAKT IN NEEMÖL ZUR BEHANDLUNG VON EPIDERMOLYSIS BULLOSA
EXTRAIT DE MILLEPERTUIS DANS DE L'HUILE DE MARGOUSIER POUR LE TRAITEMENT DE L'ÉPIDERMOLYSE BULLEUSE

(30) Priority: 24.09.2020 IT 202000022618
(43) Date of publication of application: 02.08.2023
(73) Proprietor: Ri.Mos. S.r.l., 41037 Mirandola (MO) (IT)
(72) Inventor: GORNI, Maria, 41037 MIRANDOLA (MO) (IT); LATORRE, Stefano, 00136 ROMA (IT)
(74) Representative: Negrini, Elena
(86) International application number: PCT/EP2021/075818
(87) International publication number: WO 2022/063726

(56) References cited:
- EP-A1- 2 185 168
- BRUCKNER-TUDERMAN LEENA: "Newer treatment modalities in epidermolysis bullosa", vol. 10, no. 3, 1 January 2019 (2019-01-01), pages 244, XP055803412, ISSN: 2229-5178, Retrieved from the Internet <URL:https://www.idoj.in/temp/IndianDermatolOnlineJ103244-3246524_090105.pdf> DOI: 10.4103/idoj.IDOJ_287_18

## Description

### TECHNICAL FIELD

The present invention refers to the use of a known medical-surgical device, in particular a composition comprising an extract of Hypericum in Neem oil, for use in the treatment of epidermolysis bullosa.

### PRIOR ART

It is known that the oily solution or the hypericum extract has anti-inflammatory effect, antiseptic and cicatrizing in the external wounds, ulcers and bums.

It is furthermore known that the Neem oil has cicatrizing, biocide, anti-inflammatory, myasis insect repellent and not proprieties.

For example, it is known in WO 2006/013607 that the oil mixture of Neem and the oily extract of Hypericum add the bioactive characteristics found in the single components of the mixture.

In the European patent EP 2 185 168, in the name of the Applicant, an extract of Hypericum in Neem oil, its preparation method and compositions containing said extract are described. Such compositions have cicatrizing, biocide, anti-inflammatory, myasis insect repellent proprieties.

Epidermolysis bullosa is the generic name given to a group of genetic diseases in which the skin and the lining tissues (epithelia) of the mucous membranes undergo, spontaneously or following minimal trauma, detachment and formation of bleb. The severity is very variable: there are mild forms that allow an almost normal life and very serious forms, which can be lethal in neonatal or prenatal age. The first symptoms usually appear in the first months of life. Based on the depth of the lesions, Epidermolysis bullosa is divided into three classes: simple, junctional and dystrophic. In the simple forms, the epidermis (the most superficial layer of the skin) is involved, while the mucous membranes are rarely affected; the blebs heal without scars and the main complication is the infection of the lesions. In the junctional forms, the lesions are deeper (between the epidermis and the dermis), and the blebs are extensive and often affect the mucous membranes; lethal epidermolysis bullosa (Herlitz type) belongs to this group. In dystrophic forms, the lesions are very deep and often the mucous membranes of the tongue, eyes and esophagus are involved; in the most severe forms there are malformations of the teeth and hair loss, recurrent bleeding, malnutrition and anemia.

Diagnosis is based on skin biopsy with immunofluorescence tests or transmission electron microscopy and gene analysis. Treatment is supportive and, in severe cases, specialized multidisciplinary participation is required.

There are currently no definitive treatments. Existing ones aim to prevent further complications and relieve bubble pain by means of appropriate wound treatments. Prognosis is variable, but the disease course is usually long. To date, several treatments have been tried. In children, for example, treatment with corticosteroids in combination with dapsone was found to be beneficial. Corticosteroids, dapsone, colchicine, cyclosporine, mycophenolate mofetil, IV immunoglobulins, rituximab and azathioprine have been shown to be effective in adults and people with more severe disease. Important skin care measures include gentle skin cleansing, vigilance in preventing skin trauma, and early detection and treatment of skin infections.

However, there is still a need for effective treatment of this pathology.

### SUMMARY OF THE INVENTION

We have now found that an extract of Hypericum in Neem oil, in particular the extract described in the patent EP 2 185 168 mentioned above, is particularly effective in the treatment of epidermolysis bullosa.

Therefore, the subject of the present invention is a composition containing an extract of Hypericum in Neem oil for use in the treatment of epidermolysis bullosa.

### BRIEF DESCRIPTION OF THE DRAWNINGS

The invention will be described hereinafter with the aid of the accompanying figures in which:
- figure 1 illustrates the graph of the variation of the Total Lesion Surface for the Target Lesion 1 with respect to the Baseline at the various cutpoints;
- figure 2 illustrates the graph of the variation of the Total Lesion Surface for the Target Lesion 2 with respect to the Baseline at the various cutpoints;
- figure 3 illustrates the graph of the variation of the average score of the EBDASI index with respect to the Baseline at the various cutpoints;
- figure 4 illustrates the graph of the variation of the average score of the Overall Total Score QoLEB with respect to the Baseline at the various cutpoints.

### DETAILED DESCRIPTION OF THE INVENTION

In particular, the present invention refers to an extract of Hypericum in Neem oil prepared according to the method described in patent EP 2 185 168 which includes the following steps:
- combining an amount of Hypericum flowers and/or capsules with a quantity of Neem oil with weight ratio ranging between l:3 and l:15;
- introducing into a reactor the mixture made of the amounts of Hypericum flowers and/or capsules and of Neem oil;
- maintaining the mixture vacuum, under agitation for predefined time ranging between one hour and 400 hours and temperature ranging between l6°C and l00°C.

Particularly preferred is the composition in the form of an oily solution marketed under the name Holoil in Italy and Hyperoil abroad.

The composition is administered topically.

The efficacy, tolerability and safety of the composition containing an extract of Hypericum in Neem oil for the treatment of epidermolysis bullosa has been demonstrated by means of a clinical study that will be illustrated in detail in the experimental part.

The primary end point of the study was the size reduction of the target lesions or the closure measured by imaging the lesions. Secondary end points included parameters such as: reduction or removal of skin lesion infection, assessment of the extent of skin involved in the lesion in percentage terms of total body surface area (TBSA with LUND and BROWDER Chart) at month 6, global assessment of severity indices (PGAS), assessment of disease activity indices, pain assessment using the VAS/FLACC scale and assessment of quality of life by completing the disease-specific QoLEB.

The inclusion criteria were: subjects of both sexes with a diagnosis of epidermolysis bullosa (simplex, recessive dystrophic or non-Herlitz junctional), age over two years (although a younger patient was also accepted during the study) and presence of two or more target lesions ranging in size from 5 to 250 cm² present by at least one month.

The clinical study carried out has shown that the composition containing a Hypericum extract in Neem oil is particularly effective in the treatment of epidermolysis bullosa. The composition has in fact proved to be an advanced preventive medication, as thanks to its speed and effectiveness it is possible to prevent tissue destruction at the first trace of tissue alteration.

Furthermore, it does not alter the perilesional skin and is effective in "debridement". It is atraumatic during dressing changes and reduces pain in the area of the lesion until it disappears. It allows maximum recovery of skin elasticity, ensuring the highest quality of the final scar, also reducing the formation of keloids and eschar. Its use also prevents recurrences.

### Experimental part

The clinical study was performed on 10 patients aged 16 months to 32 years diagnosed with recessive dystrophic epidermolysis.

The oily solution containing Hypericum extract in Neem oil (Holoil) was administered topically to patients for a period of 6 months with follow-up visits before the start of treatment and after 30 ± 5, 90 ± 5 and 180 ± 5 days of treatment.

Of the 10 enrolled patients, follow-up is only available for 9 with the following results:
- 5 patients had lesion resolution within 197 days
- 3 patients showed a reduction of the lesion, with a percentage reduction with respect to the baseline respectively equal to:
   - 7% in 214 days
   - 28% in 43 days
   - 38% in 210 days
   - 1 patient with sepsis.

The average value of the Total Lesion Surface (cm²) for Target Lesion 1 showed a decrease between the baseline evaluation (54,24 cm²) and the last evaluation (22,11 cm²) equal to 59,2%.

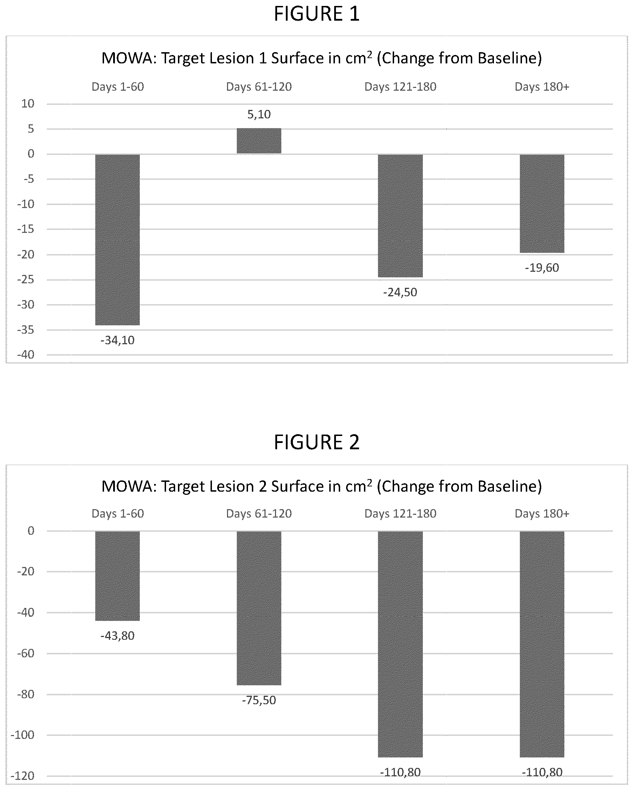

Figure 1 shows the graph of the variation with respect to the Baseline at the various cutpoints (0 taken as a reference for the Baseline).

The average value of the Total Lesion Surface (cm²) for the Target Lesion 2 showed a decrease between the baseline evaluation (64,27 cm²) and the last evaluation (4,16 cm²) equal to 93,5%.

Figure 2 shows the graph of the variation with respect to the Baseline at the various cutpoints (0 taken as a reference for the Baseline).

At the first visit, the PGAS (Physician Global Assessment of Severity) score for Lesion 1 is shown in the following table:

| **Target Lesion 1 Category** | **Freq.** | **Percent** | **Cum.** |
|---|---|---|---|
| 1-2 - (Almost Clear) - (infrequent blistering/erosion) | 1 | 10.00 | 10.00 |
| 3-4 - (Mild disease) - (up to 15% of body affected) | 5 | 50.00 | 60.00 |
| 5-6 - (Moderate disease) - (between 16-25% of body affected) | 2 | 20.00 | 80.00 |
| 7-8 - (Severe disease) - (between 26-50% of body affected) | 2 | 20.00 | 100.00 |
| Total | 10 | | |

In the three patients with lesion reduction, the following changes are observed between first and last evaluation:

| **First Visit** | **Last Visit** |
|---|---|
| 3-4 - (Mild disease) - (up to 15% of body affected) | 3-4 - (Mild disease) - (up to 15% of body affected) |
| 7-8 - (Severe disease) - (between 26-50% of body affected) | 5-6 - (Moderate disease) - (between 16-25% of body affected) |
| 5-6 - (Moderate disease) - (between 16-25% of body affected) | 3-4 - (Mild disease) - (up to 15% of body affected) |

At the first examination, the PGAS score is equal to:

| **Target Lesion 2 Category** | **Freq.** | **Percent** | **Cum.** |
|---|---|---|---|
| 3-4 - (Mild disease) - (up to 15% of body affected) | 7 | 70.00 | 70.00 |
| 5-6 - (Moderate disease) - (between 16-25% of body affected) | 2 | 20.00 | 90.00 |
| 7-8 - (Severe disease) - (between 26-50% of body affected) | 1 | 10.00 | 100.00 |
| Total | 10 | | |

At the last evaluation, all the second target lesions appeared in the state:

### 0- (clear) - (no blistering/erosions)

The average score of the EBDASI index shows a decrease between the baseline evaluation (101,7) and the last evaluation (81,9) equal to 19,5%.

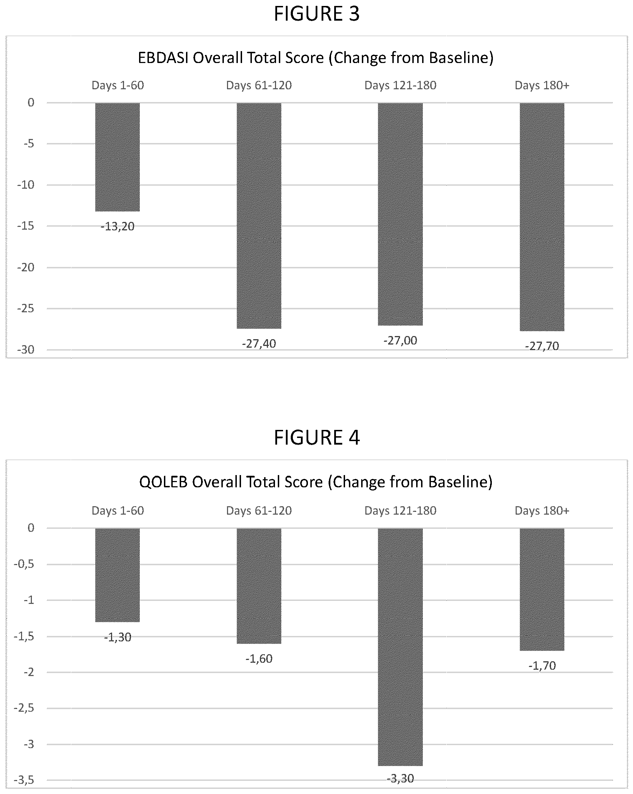

Figure 3 shows the graph of the variation with respect to the Baseline at the various cutpoints (0 taken as a reference for the Baseline).

The average score of the Overall Total Score QoLEB shows a slight decrease between the evaluation at the Baseline (25,3) and the last evaluation (23,0) equal to 9,1%.

Figure 4 shows the graph of the variation with respect to the Baseline at the various cutpoints (0 taken as a reference for the Baseline).

### FLACC Scale

the FLACC scale is indicated for only two patients:
- 1 patient: reduction from 3 to 0
- 1 patient: reduction from 4 to 1

Average reduction of three points:

### VAS Scale

For the seven patients for whom the VAS scale was measured, there was a constant decrease over time with an average score of 13,3 at the Baseline and 7,4 at the last evaluation, with a decrease equal to 44,4%.

The above data demonstrate the efficacy of Hypericum extract in Neem oil in the treatment of epidermolysis bullosa.

## Claims

1. Composition containing Hypericum extract in Neem oil for the use in the treatment of Epidermolysis Bullosa.

2. Composition for use in the treatment of Epidermolysis Bullosa according to claim 1 wherein the Hypericum extract in Neem oil is carried out by means of a method including the steps of:
- combining an amount of Hypericum flowers and/or capsules with a quantity of Neem oil with weight ratio ranging between 1:3 and 1: 15;
- introducing into a reactor the mixture comprising the amounts of Hypericum flowers and/or capsules and Neem oil;
- maintaining the mixture vacuum, under agitation for predefined time ranging between one hour and 400 hours and temperature ranging between 16°C and 100°C.

3. Composition for use in the treatment of Epidermolysis Bullosa according to claim 1 or claim 2 as oily solution.

4. Composition for use in the treatment of Epidermolysis Bullosa according to anyone of the preceding claims for topical administration.

## Patentansprüche

1. Zusammensetzung enthaltend Hyperikumextrakt in Neemöl zur Verwendung bei der Behandlung von Epidermolysis Bullosa.

2. Zusammensetzung zur Verwendung bei der Behandlung von Epidermolysis Bullosa gemäß Anspruch 1, wobei das Hyperikumextrakt in Neemöl gewonnen wird mittels eines Verfahrens umfassend die Schritte:
- Kombinieren einer Menge von Hyperikumblüten und/oder -kapseln mit einer Menge von Neemöl mit einem Gewichtsverhältnis im Bereich zwischen 1:3 und 1:15;
- Einleiten der Mischung bestehend aus den Mengen von Hyperikumblüten und/oder -kapseln und Neemöl in einen Reaktor;
- Beibehalten des Mischvakuums, unter Rühren über eine im Voraus bestimmte Zeit im Bereich zwischen einer Stunde und 400 Stunden und einer Temperatur zwischen 16°C und 100°C.

3. Zusammensetzung zur Verwendung bei der Behandlung von Epidermolysis Bullosa gemäß Anspruch 1 oder Anspruch 2 als ölige Lösung.

4. Zusammensetzung zur Verwendung bei der Behandlung von Epidermolysis Bullosa gemäß einem der vorhergehenden Ansprüche zur topischen/lokalen Anwendung.

## Revendications

1. - Composition contenant un extrait de millepertuis dans de l'huile de margousier pour l'utilisation dans le traitement de l'épidermolyse huileuse.

2. - Composition pour l'utilisation dans le traitement de l'épidermolyse bulleuse selon la revendication 1, dans laquelle l'extrait de millpertuis dans l'huile de margousier est réalisé au moyen d'un procédé comprenant les étapes consistant à :
- combiner une quantité de fleurs et/ou de gélules de millepertuis avec une quantité d'huile de margousier avec un rapport pondéral se situant entre 1:3 et 1:15 ;
- introduire dans un réacteur le mélange comprenant les quantités de fleurs et/ou de gélules de millepertuis et d'huile de margousier ;
- maintenir le mélange sous vide, sous agitation pendant une durée prédéfinie se situant entre une heure et 400 heures et une température se situant entre 16°C et 100°C.

3. - Composition pour l'utilisation dans le traitement de l'épidermolyse bulleuse selon la revendication 1 ou la revendication 2 sous forme de solution huileuse.

4. - Composition pour l'utilisation dans le traitement de l'épidermolyse bulleuse selon l'une quelconque des revendications précédentes pour une administration topique.
